# EUROPEAN PATENT APPLICATION

(11) **EP 4 327 792 A1**
(43) Date of publication of application: **28.02.2024**
(21) Application number: 23179136.9
(22) Date of filing: 14.06.2023
(51) Int. Cl.: A61F 13/49, A61F 13/505, A61F 13/539

(54) **UNDERGARMENT HAVING REMOVABLE ABSORBENT LINER**

(30) Priority: 23.08.2022 US 202217893711
(71) Applicant: VBrite, LLC, Bentonville, AR 72712 (US)
(72) Inventor: LIOU, Shyhshin, Bentonville, 72712 (US)
(74) Representative: Becker, Eberhard

(57) **Abstract**

An undergarment for covering a lower trunk has a clothing (10) and an absorbent liner (20). The clothing (10) is configured to cover the lower trunk and has a first adhering layer (11) formed on a crotch portion of the clothing (10). The absorbent liner (20) removably adheres to the crotch portion of the clothing (10) and is configured to be disposed between the clothing (10) and the lower trunk. The absorbent liner (20) has a second adhering layer (21) and a skin-contacting layer (22). The second adhering layer (21) removably adheres to the first adhering layer (11) of the clothing (10), and the skin-contacting layer (22) is configured to contact crotch of the lower trunk directly. When the absorbent liner (20) becomes full, only the absorbent liner (20) itself has to be replaced, making the undergarment more convenient to use.

## Description

### 1. Field of the Invention

The present invention relates to underwear, and especially relates to underwear utilizing friction between fabrics to attach a removable pad to a main clothing of the underwear. The use of the underwear can be for males or females, adults or kids, humans or animals.

### 2. Description of the Prior Arts

Disposable products such as tampons or sanitary pads are often used to solve physical or mental discomfort resulting from discharges such as the menstrual blood during menstrual period, urine leakage from incontinence, or discharges from yeast or bacterial infection. However, the use of one time use disposable products has raised environmental pollution concerns. It is also difficult to predict the timing and volume of the discharges, as well as how to keep the underwear clean over a period of time and avoid leakage from excessive discharges or disposable product failure.

The commonly called period or leakproof panty with absorbent and leakproof layers sewn directly into the panty itself has been made available commercially. However, when the absorbent area needs to be replaced, the panty and outerwear such as pants, socks and shoes may need to be completely removed in order to change for a new panty. This operation can create a lot of inconvenience when people are in public. Changing underwear may be a process that people need to take off their pants, shoes and socks to avoid cross contamination from blood, urine or any other body discharges to their outer apparel. As a result, the wearer has to carry the whole used panty with her/him until there is a place where the used panty can be changed or washed, and therefore, this is still very inconvenient.

There are some individual fabric pad solutions in which a fabric pad is glued, buttoned, snapped, tied, or inserted into a pocket on the underwear. However, all existing solutions require additional add-ons such as glue or buttons, and therefore result in uncomfortable or insecure fit due to the additional add-ons to attach the fabric pad to the underwear.

To overcome the shortcomings, the present invention provides an undergarment having a removable fabric liner with a structure similar to a hook-and-loop fastener to mitigate or obviate the aforementioned problems.

The main objective of the present invention is to use fabric's natural resistance to lock the liner pad with the undergarment fabric to make easier the replacing of the reusable fabric liner pad.

The undergarment for covering a lower trunk has a clothing and an absorbent liner. The clothing is configured to cover the lower trunk and has a first adhering layer formed on a crotch portion of the clothing. The first adhering layer is a woven fabric and has a first fuzz structure. The absorbent liner removably adheres to the crotch portion of the clothing and is configured to be disposed between the clothing and the lower trunk. The absorbent liner has a second adhering layer and a skin-contacting layer. The second adhering layer is a woven fabric and has a second fuzz structure. The skin-contacting layer is configured to contact crotch of the lower trunk directly. The second fuzz structure of the absorbent liner removably engages with the first fuzz structure of the clothing to make the absorbent liner removably adhere to the crotch portion of the clothing.

The absorbent liner is configured to absorb period blood, bladder leaks, and pregnancy leaks or bodily discharge. When the absorbent liner becomes full or needs to be replaced, it can be easily removed from the crotch area for washing, and a new absorbent liner can be adhered to the clothing without glue, button, snap, tie, or insert to a pocket on the clothing.

The advantage of the present invention is that when the absorbent liner becomes full, only the absorbent liner itself has to be replaced; that is, instead of the whole used undergarment, the wearer only has to carry the used absorbent liner, making the present invention more convenient. The use of reusable fabric liner pad with the underwear will also help to reduce the waste from disposable liner pad or tampon. Moreover, as the first and second adhering layers are woven fabrics, the undergarment requires no additional add-ons to attach the absorbent liner to the clothing, and is therefore more comfortable.

In other words, the present invention has removable absorbent pad fabric fastened with an underwear crotch fabric using a hook-and-loop kind of design. The combination of the removable absorbent pad and the clothing is used as a washable, reusable leak-resistant underwear solution for liquid such as blood, urine or other body discharges. The two fabrics, one on the back of the pad and the other on the crotch area of the clothing, are made like a hook-and-loop fastening structure to securely attach the absorbent pad on the crotch area of the clothing but still easily removable.

The fastening structure between the washable and reusable absorbent fabric pad and the clothing does not require any additional add-ons such as glue, button, snap, string tie, or pockets as the fastening method. The clothing and the removable pads can be used in different combinations in terms of sizes, shapes, and fabric materials for different absorption or leakage prevention needs. The clothing and pad can be used for males or females, adults or kids, humans or animals.

Other objectives, advantages and novel features of the invention will become more apparent from the following detailed description when taken in conjunction with the accompanying drawings.

### IN THE DRAWINGS

Fig. 1 is a perspective view of an undergarment in accordance with the present invention;
Fig. 2 is an enlarged perspective view of the undergarment in Fig. 1;
Fig. 3 is an enlarged exploded perspective view of the undergarment in Fig. 1;
Fig. 4 is another enlarged perspective view of the undergarment in Fig. 1, showing an absorbent liner being partially removed from a clothing;
Fig. 5 is an enlarged sectional view of the undergarment in Fig. 4;
Fig. 6 is an enlarged schematic sectional view of the undergarment in Fig. 1, showing a second fuzz structure of a second adhering layer and a first fuzz structure of a first adhering layer before adhering together;
Fig. 7 is an enlarged schematic perspective view of another embodiment of the second fuzz structure and the first fuzz structure; and
Fig. 8 is an enlarged schematic perspective view of still another embodiment of the first fuzz structure.

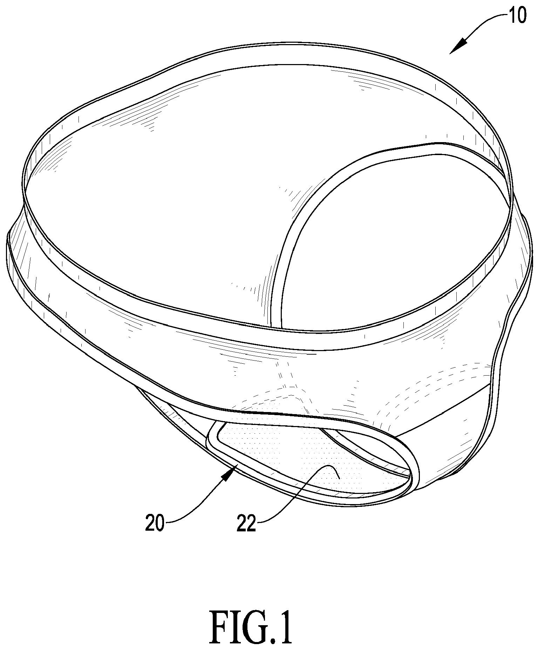

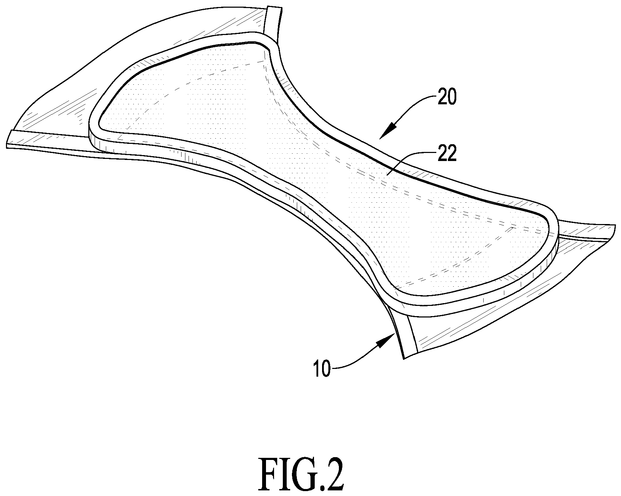

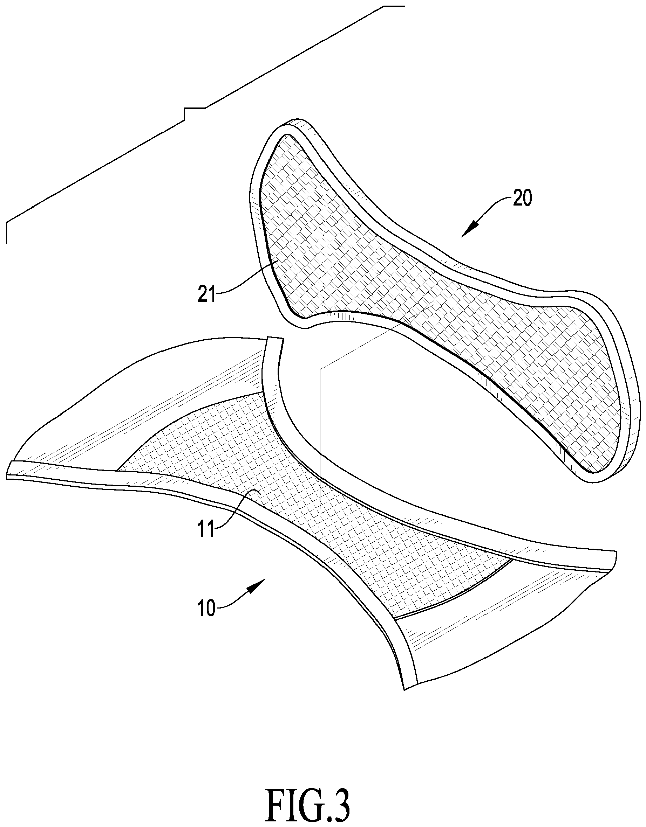

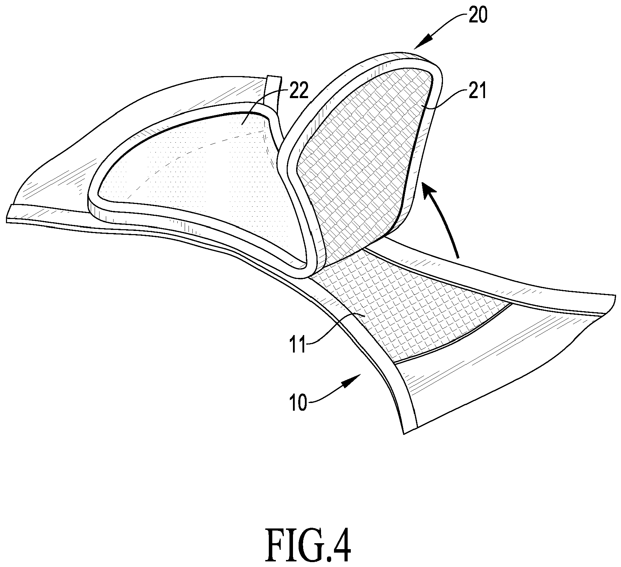

With reference to Figs. 1 to 5, an undergarment in accordance with the present invention is for covering a lower trunk of a wearer, and is preferably a leakproof panty. The undergarment comprises a clothing 10 and an absorbent liner 20, and both the clothing 10 and the absorbent liner 20 are preferably washable/reusable.

The clothing 10 is configured to cover the lower trunk and has a first adhering layer 11 formed on a crotch portion of the clothing. The first adhering layer 11 is self-adhesive similar to a hook-and-loop fastener, but has no visible hook or loops; that is, the first adhering layer is smooth and comfortable when in direct contact with skin, and therefore the clothing 10 is comfortably worn with or without the absorbent liner 20. The self-adhesive property of the first adhering layer 11 is durable after multiple uses and water washes.

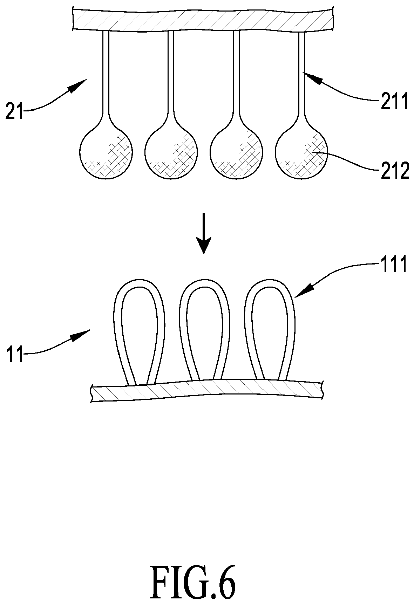

With reference to Fig. 6, the first adhering layer 11 is preferably textile, and is preferably woven fabric. The self-adhesive property of the first adhering layer is provided by a first fuzz structure 111 formed on a surface of the first adhering layer 11. Said first fuzz structure 111 is configured in a way that the first fuzz structure 111 is capable of removably adhering to another specifically designed fuzz structure. In the preferred embodiment, the first fuzz structure 111 has miniature loops for adhering.

With reference to Figs. 1 to 5, the absorbent liner 20 removably adheres to the crotch portion of the clothing 10 and is configured to be disposed between the clothing 10 and the lower trunk; that is, the absorbent liner 20 is configured to be in direct contact with the wearer.

The absorbent liner 20 has a second adhering layer 21 and a skin-contacting layer 22. The second adhering layer 21 removably adheres to the first adhering layer 11 of the clothing 10, and the skin-contacting layer 22 is configured to contact the crotch of the wearer directly. In the preferred embodiment, an enhanced absorbent layer 23 and a leak-resistant layer 24 are disposed between the second adhering layer 21 and the skin-contacting layer 22.

The second adhering layer 21 is self-adhesive like the first adhering layer 11, and the self-adhesive property of the second adhering layer 21 is also durable after multiple uses and water washes.

With reference to Fig. 6, the second adhering layer 21 is preferably textile, and is preferably woven fabric. The self-adhesive property of the second adhering layer 21 is provided by a second fuzz structure 211 formed on a surface of the second adhering layer 21. The second fuzz structure 211 of the second adhering layer 21 removably engages with the first fuzz structure 111 of the first adhering layer 11 to removably adhere the second adhering layer 21 to the first adhering layer 11 and also generates sufficient friction to hold the absorbent liner 20 in place.

In the preferred embodiment, the second fuzz structure 211 of the second adhering layer 21 has miniature fuzz protrusions which are configured to removably engage with the miniature loops of the first fuzz structure 111. To be precise, each of the miniature fuzz protrusions of the second fuzz structure 211 has an enlarged head portion 212 which can be pushed through one of the miniature loops of the first fuzz structure 111 such that the enlarged head portion 212 is surrounded and tightened by the miniature loop; therefore, the second fuzz structure 211 is capable of removably engaging with the first fuzz structure 111.

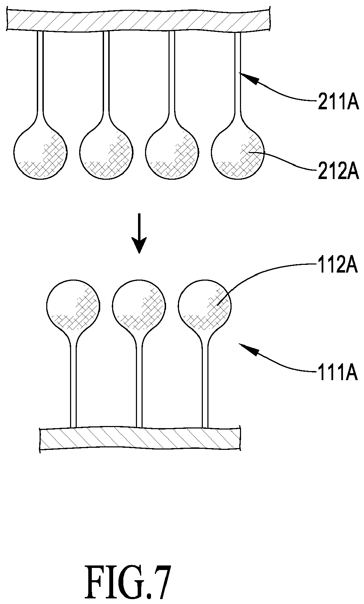

With reference to Fig. 7, in another preferred embodiment, the miniature loops of the first fuzz structure 111A are replaced by miniature fuzz protrusions with enlarged head portions 112A. The enlarged head portions 212A of the second fuzz structure 211A can be clamped between the enlarged head portions 112A of the first fuzz structure 111A such that the second fuzz structure 211A is capable of removably engaging with the first fuzz structure 111A.

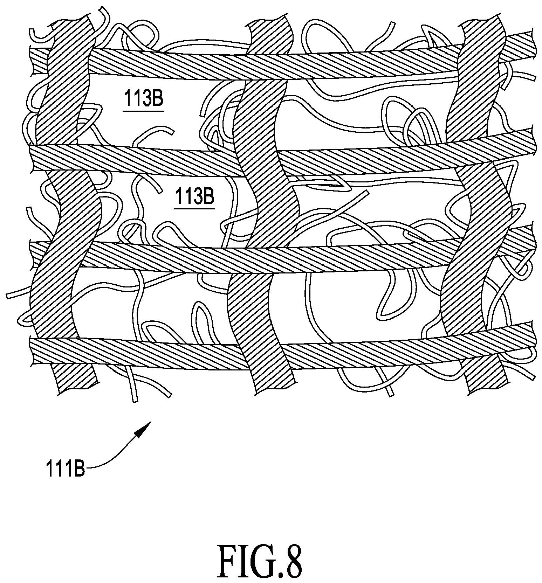

With reference to Fig. 8, in still another preferred embodiment, the first fuzz structure 111B of the first adhering layer is a mesh-type structure forming multiple engaging holes 113B. Each of the enlarged head portions 212 of the second fuzz structure 211 can be pushed through one of the engaging holes 113B of the first fuzz structure 111 such that the second fuzz structure 211 is capable of removably engaging with the first fuzz structure 111B.

Figs. 6 to 8 are only showing preferred embodiments of the first fuzz structure 111 and the second fuzz structure 211, and scope of the first and second fuzz structures is by no means limited by Figs. 6 to 8. The scope of the first and second fuzz structures is at least as broad as given by the claims.

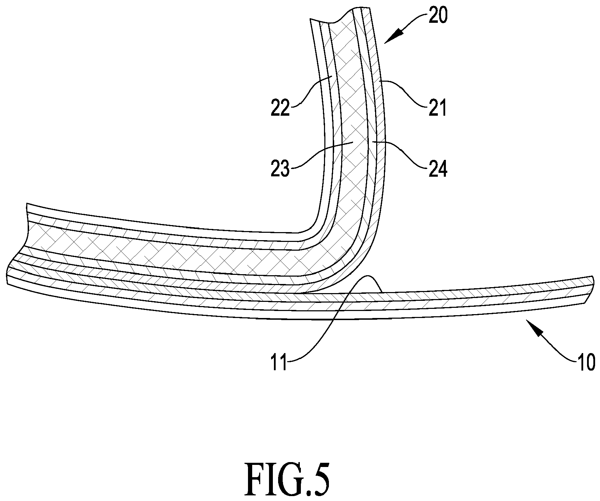

With reference to Fig. 5, the skin-contacting layer 22 is preferably made of bacteria-resistant fabric. The leak-resistant layer 24 is adjacent to the second adhering layer 21, water-resistant, and preferably breathable.

The undergarment is worn like a conventional period panty, but the difference is that when the absorbent liner 20 is full, it can be easily removed by pulling, and a new absorbent liner 20 can be adhered to the clothing 10 such that the clothing 10 does not have to be replaced together with the absorbent liner 20.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. An undergarment, **characterized in that** the undergarment is for covering a lower trunk and comprises:
a clothing (10) configured to cover the lower trunk and having:
a first adhering layer (11) formed on a crotch portion of the clothing (10); the first adhering layer (11) being a woven fabric and having a first fuzz structure (111); and
an absorbent liner (20) removably adhering to the crotch portion of the clothing (10) and configured to be disposed between the clothing (10) and the lower trunk; the absorbent liner (20) having:
a second adhering layer (21) being a woven fabric and having a second fuzz structure (211); and
a skin-contacting layer (22) configured to contact a crotch of the lower trunk directly;
wherein the second fuzz structure (211) of the absorbent liner (20) removably engages with the first fuzz structure (111) of the clothing (10) to make the absorbent liner (20) removably adhere to the crotch portion of the clothing (10).

2. The undergarment as claimed in claim 1, wherein the absorbent liner (20) has a leak-resistant layer (24) disposed between the second adhering layer (21) and the skin-contacting layer (22).

3. The undergarment as claimed in claim 1, wherein the absorbent liner (20) has an enhanced absorbent layer (23) and a leak-resistant layer (24); the enhanced absorbent layer (23) and the leak-resistant layer (24) are disposed between the second adhering layer (21) and the skin-contacting layer (22).
